(19)

Europäisches
Patentamt
European
Patent Office
Office européen
des brevets

(11) **EP 2 567 719 A2**

(12) **EUROPEAN PATENT APPLICATION**
published in accordance with Art. 153(4) EPC

(43) Date of publication:
**13.03.2013 Bulletin 2013/11**

(21) Application number: **11777594.0**

(22) Date of filing: **06.05.2011**

(51) Int Cl.:
*A61M 5/00* (2006.01)        *A61M 5/168* (2006.01)
*G06Q 50/00* (2012.01)

(86) International application number:
**PCT/KR2011/003384**

(87) International publication number:
**WO 2011/139113 (10.11.2011 Gazette 2011/45)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.05.2010   KR 20100042523**

(71) Applicant: **Woo Young Medical Co., Ltd.
Jincheon-gun, Chungcheongbuk-do 365-801
(KR)**

(72) Inventor: **LEE, Young Gyu
Seoul 135-280 (KR)**

(74) Representative: **Mammel und Maser
Patentanwälte
Tilsiter Straße 3
71065 Sindelfingen (DE)**

(54) **METHOD FOR MANAGING HISTORY OF LIQUID MEDICINE INJECTIONS**

(57)    The present invention relates to a method of managing a history of the injection of a medicinal fluid. More particularly, the present invention relates to a method of managing a history of the injection of a medicinal fluid, which is capable of managing the history of the injection of a medicinal fluid in an apparatus for injecting a medicinal fluid that automatically injects a predetermined volume of medicinal fluid into a vein or epidural space of a patient in order to manage the pain of the patient.

[FIG. 2]

## Description

### Technical Field

The present invention relates to a method of managing a history of the injection of a medicinal fluid. More particularly, the present invention relates to a method of managing a history of the injection of a medicinal fluid, which is capable of managing the history of the injection of a medicinal fluid in an apparatus for injecting a medicinal fluid that automatically injects a predetermined volume of medicinal fluid into a vein or epidural space of a patient in order to manage the pain of the patient.

### Background Art

In general, when a medicinal fluid, including a drug, is injected into a patient in a hospital or the like in order to cure a patient or relieve pain, a predetermined volume of medicinal fluid is injected into the patient by fixing a medicinal fluid pack at a location above a syringe inserted into the patient and then manipulating a value capable of regulating the speed at which the medicinal fluid is injected.

However, when the above method is used, an intermittent spasm or pain may occur in a patient into which the medicinal fluid is being injected. In the case in which the volume of the medicinal fluid to be injected is regulated by a patient when it is necessary to increase the volume of the medicinal fluid to be injected, the state of the patient may be jeopardized. Accordingly, an additional prescription is obtained and then the volume of medicinal fluid to be injected is regulated, so that there arise the problems of a long time being required and an increased burden being imposed on a doctor or a nurse.

Furthermore, the method is problematic in that it is not easy to perform regulation so that a predetermined volume of medicinal fluid can be injected into a patient over time in accordance with a prescription because the injection of the medicinal fluid is manually regulated, in that an individual should carry the medicine around because in the case of a patient who requires the injection of a medicinal fluid only in specific situations because of a disease such as an acute cardiac seizure, the rapid injection of medicine is required in the specific situations, and in that a user should be skilled at making accurate injections because an accurate volume of medicinal fluid should be injected upon administering the injection.

Furthermore, the above method is problematic in that the safety of a patient cannot be guaranteed because a user may erroneously inject an excessive volume of medicinal fluid into the patient even when a user is skilled at injecting a medicinal fluid, and in that it is very difficult for a patient to deal with unexpected situations such as a case in which impurities or air are included in a medicinal fluid or a medicinal fluid pack is pressed.

Accordingly, in order to solve the above problems, an apparatus for injecting a medicinal fluid, which is capable of operating in the same manner as a pump and injecting a medicinal fluid, including an antibiotic or an anticancer drug, into a patient with a serious disease such as cancer or leukemia in minute amounts over a long stretch of time, or capable of enabling a patient to administer an analgesic whenever pain continuously or intermittently occurs has been developed and used.

However, the conventional apparatus for injecting a medicinal fluid has the disadvantage of not supporting the systematic management of the state of a patient with a serious disease or a patient who continuously has pain after undergoing an operation because a medicinal fluid is simply injected into a patient according to preset information and a history of the injection of the medicinal fluid is not managed in the apparatus for injecting a medicinal fluid.

Furthermore, the conventional apparatus for injecting a medicinal fluid is problematic in that it is not easy to check on input setting information so as to detect a setting error that may have occurred during the input of the setting information input and thus a history of the injection of a medicinal fluid cannot be systematically managed because the setting information cannot be displayed on a single screen but is displayed in a stepwise manner and thereafter a medicinal fluid is injected according to the setting information.

### Disclosure

### Technical Problem

The present invention has been made keeping in mind the above problems occurring in the prior art, and an object of the present invention is to provide a method of managing a history of the injection of a medicinal fluid, which can manage a history of the injection of a medicinal fluid being injected into a patient, so that the state of the injection of the medicinal fluid into a patient can be checked in real time and also the speed at which the medicinal fluid is injected into the patient can be regulated depending on the state of the injection of the medicinal fluid.

**Technical Solution**

In order to accomplish the above object, the present invention provides a method of managing a history of the injection of a medicinal fluid in an apparatus for injecting a medicinal fluid, the method including step (a) of setting information about injection of a medicinal fluid at an apparatus for injecting a medicinal fluid; step (b) of storing a history of injection of the medicinal fluid into a patient based on the set information about injection of the medicinal fluid for a predetermined time interval; and step (c) of analyzing the stored history of injection of the medicinal fluid for the predetermined time interval, and regulating a state of injection of the medicinal fluid into the patient.

The information about injection of the medicinal fluid of step (a) may include the total volume of the medicinal fluid, the flow rate of the medicinal fluid, the amount of drug included in the medicinal fluid, a bolus dose, a bolus rate, and a bolus limit per time.

The method may further include, after step (a), step (a1) of displaying the information about injection of the medicinal fluid.

Step (a1) may include step (a11) of displaying the total volume of the medicinal fluid and the flow rate of the medicinal fluid; step (a12) of displaying the drug amount, computing a drug rate using the total volume of the medicinal fluid, the flow rate of the medicinal fluid, and the drug amount, and displaying the drug rate; and step (a13) of displaying the bolus dose and the bolus rate.

The drug rate of step (a12) may be computed using the following Equation:

$$D_R = (D_A/T_V)*F_R$$

where $D_R$ is the drug rate, $D_A$ is the drug amount, $T_V$ is the total volume of the medicinal fluid, and the $F_R$ is the flow rate of the medicinal fluid.

Step (b) may include step (b1) of checking a volume of an injected medicinal fluid for the predetermined time interval, and storing the volume of the injected medicinal fluid and the total volume of the medicinal fluid for the predetermined time interval; and step (b2) of checking a bolus attempt count and a bolus injection count for the predetermined time interval, and storing the bolus attempt count, the bolus injection count, and the bolus dose.

The method may further include, after step (b2), step (b3) of checking whether the apparatus for injecting a medicinal fluid was temporarily stopped during the predetermined time interval, and storing a history of the temporary stopping of the apparatus for injecting a medicinal fluid for the predetermined time interval.

The volume of the injected medicinal fluid for the predetermined time interval and total volume of the medicinal fluid of step (b1), the bolus attempt count, bolus injection count and bolus dose of step (b2), and the history of the temporary stopping of step (b3) may be displayed on the apparatus for injecting a medicinal fluid.

Step (c) may include step (c1) of computing a volume of shortage of the injected medicinal fluid for the predetermined time interval using the total volume of the medicinal fluid and the volume of the injected medicinal fluid stored for the predetermined time interval; and step (c2) of regulating a speed at which the medicinal fluid is injected so that a medicinal fluid corresponding to the computed volume of the shortage of the injected medicinal fluid can be injected within a subsequent predetermined time interval.

The method may further include, after step (c2), step (c3) of computing a difference between the bolus attempt count and the bolus injection count stored for the predetermined time interval, and regulating a bolus dose for subsequent bolus injection if the difference is equal to or higher than a predetermined reference value.

**Advantageous Effects**

The present invention has the advantage of actively dealing with the state of a patient because information about the injection of a medicinal fluid can be set in the apparatus for injecting a medicinal fluid and then a history of the injection of a medicinal fluid injected into the patient based on the set information about the injection of the medicinal fluid can be stored for each predetermined time interval or the stored history of the injection of the medicinal fluid can be displayed on the screen display unit, and because the stored history of the injection of the medicinal fluid can be analyzed and then the state of the injection of the medicinal fluid into the patient can be regulated.

Furthermore, the present invention has the advantage of rapidly monitoring the state of a patient, into whom a medicinal fluid is being injected by an apparatus for injecting a medicinal fluid, in real time because information about the injection of the medicinal fluid and the history of the injection of the medicinal fluid can be displayed.

Furthermore, the present invention has the advantage of reducing errors that may occur in a process of setting information about the injection of a medicinal fluid, compared to a conventional stepwise display method, because information about the injection of the medicinal fluid and the history of the injection of the medicinal fluid can be displayed on a single screen at the same time.

**Description of Drawings**

FIG. 1 is a perspective view of an apparatus for injecting a medicinal fluid according to a preferred embodiment of the present invention;

FIG. 2 is a flowchart of a method of managing a history of the injection of a medicinal fluid according to a preferred embodiment of the present invention;

FIG. 3 is a detailed flowchart of step S20 of FIG. 2;

FIG. 4 is a reference diagram of the display of information about the injection of a medicinal fluid according to a preferred embodiment of the present invention;

FIG. 5 is a detailed flowchart of step S30 of FIG. 2;

FIG. 6 of a reference screen of the display of general information about the injection of a medicinal fluid according to a preferred embodiment of the present invention;

FIG. 7 is a reference screen of the display of a bolus history according to a preferred embodiment of the present invention;

FIG. 8 is a reference screen of a history of the stopping of the display apparatus for injecting a medicinal fluid according to a preferred embodiment of the present invention; and

FIG. 9 is a detailed flowchart of step S40 of FIG. 2.

**Best Mode**

Embodiments of the present invention will be described in detail below with reference to the accompanying drawings. It should be noted that when reference numerals are assigned to the elements of the drawings, the same reference numeral is assigned to the same elements even when they have been illustrated in different drawings. Furthermore, in the following description of the present invention, detailed descriptions of related known configurations and/or functions will be omitted if they are deemed to make the gist of the present invention unnecessarily vague. Furthermore, although preferred embodiments of the present invention will be described, it will be apparent that the technical spirit of the present invention can be practiced by those skilled in the art without being restricted or limited to the preferred embodiments.

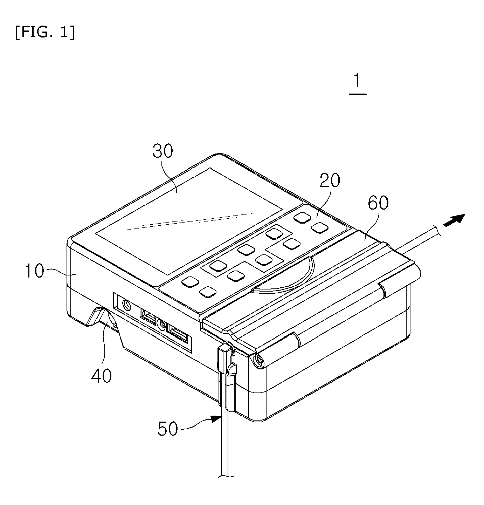

FIG. 1 is a perspective view of an apparatus for injecting a medicinal fluid according to a preferred embodiment of the present invention.

As shown in FIG. 1, the apparatus 1 for injecting a medicinal fluid according to the preferred embodiment of the present invention includes a body unit 10, a manipulation unit 20, a screen display unit 30, a bolus switch connection socket 40, a medicinal fluid injection tube 50, and a door unit 60.

The manipulation unit 20 is located on the front of the body unit 10. Information about the injection of a medicinal fluid is set by manipulating the manipulation unit 20 so as to inject the medicinal fluid into a patient.

Here, the information about the injection of a medicinal fluid may include the total volume of the medicinal fluid, the flow rate of the medicinal fluid, the amount of drug included in a medicinal fluid, the bolus dose, the bolus rate, and the bolus limit per time.

Furthermore, the information about the injection of a medicinal fluid may further include a medicinal fluid injection mode. The medicinal fluid injection mode may be a continuous mode in which a predetermined volume of medicinal fluid is continuously injected during an operating period from the initiation of the injection of a medicinal fluid to the termination thereof, a patent-controlled analgesia (PCA) mode in which a specific volume of medicinal fluid is injected in addition to the predetermined volume of medicinal fluid injected in the continuous mode, and a patient-controlled epidural analgesia (PCEA) mode which is similar to the PCA mode and in which the medicinal fluid is injected not via a vein but via an epidural space.

Furthermore, the information about the injection of a medicinal fluid may further include the units (mg, $\mu$g, or

mL) of the drug included in the medicinal fluid, the LOT which is the time which ranges from the injection of a bolus up to the subsequent injection of a bolus and for which the bolus switch is locked, the LD which is the dose of bolus that is administered to a patient at the beginning of the injection of the medicinal fluid, the bolus rate, and the KVO rate that is intended to keep a vein open even after the completion of the injection of the medicinal fluid.

The screen display unit 30 is located above the manipulation unit 20, and displays the information about the injection of a medicinal fluid that is set via the manipulation unit 20.

A bolus switch (not shown) that is manipulated to temporarily increase the volume of the medicinal fluid to be injected into the patient when the patient feels pain during the injection of the medicinal fluid based on the information about the injection of a medicinal fluid is connected to the bolus switch connection socket 40.

Here, the increase in the volume of the medicinal fluid to be injected into the patient, which is performed by the manipulation of the bolus switch, may be performed based on the information about the injection of a medicinal fluid set via the manipulation unit 20 prior to the injection of the medicinal fluid into the patient, that is, the bolus dose, the bolus rate, the bolus limit per time, the LOT, and the LD.

The medicinal fluid injection tube 50 is connected at its one end to a medicinal fluid pack (not shown) outside the apparatus 1 for injecting a medicinal fluid, and is connected at its other end to the patient. The medicinal fluid injection tube 50 is inserted and coupled to a portion below the connected manipulation unit 20, and acts as a tube that carries the medicinal fluid from the medicinal fluid-filled pack to the patient in response to the operation of the apparatus 1 for injecting a medicinal fluid.

The door unit 60 is located in front of the medicinal fluid injection tube 50 so that it can be selectively opened and closed, and allows the medicinal fluid injection tube 50 to be securely seated and accommodated therein. The apparatus 1 for injecting a medicinal fluid is operated such that the medicinal fluid can be injected into the patient via the medicinal fluid injection tube 50 only after the medicinal fluid injection tube 50 has been securely seated by the door unit 60.

Here, the medicinal fluid injection tube 50 may be operated by a driving unit (not shown) that is located in the lower portion of the apparatus 1 for injecting a medicinal fluid and that pumps the medicinal fluid through the medicinal fluid injection tube 50.

Furthermore, the apparatus 1 for injecting a medicinal fluid according to the preferred embodiment of the present invention may further include a door sensor for detecting the opening and closing of the door unit 60d that allows the medicinal fluid injection tube 50 to be securely seated and accommodated, and a blockage sensor for detecting the state of the blockage of the medicinal fluid injection tube 50.

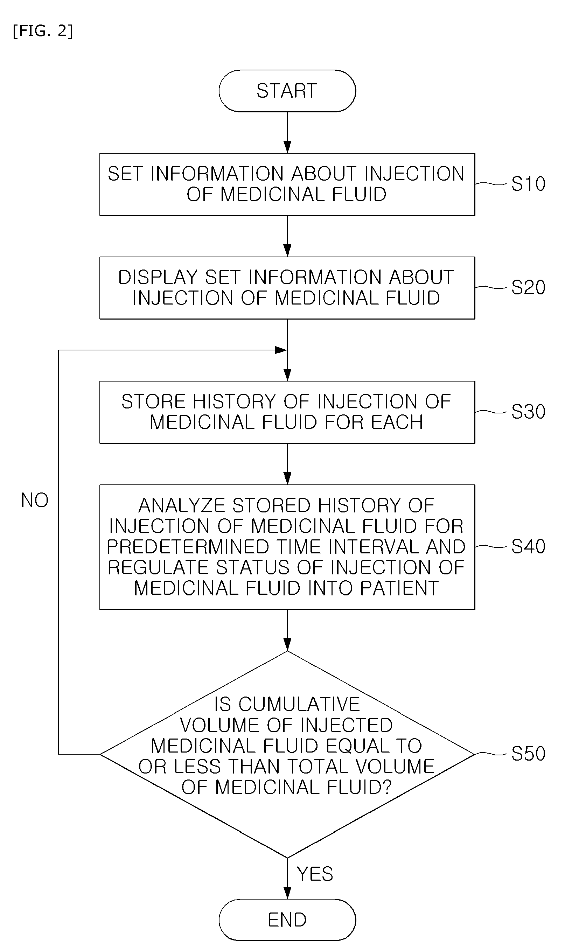

FIG. 2 is a flowchart of a method of managing a history of the injection of a medicinal fluid according to a preferred embodiment of the present invention.

As shown in FIG. 2, at step S10, information about the injection of a medicinal fluid is set by manipulating the manipulation unit 20 of the apparatus 1 for injecting a medicinal fluid.

Here, the information about the injection of a medicinal fluid may include the total volume of the medicinal fluid, the flow rate of the medicinal fluid, the amount of drug included in the medicinal fluid, the bolus dose, the bolus rate, and the bolus limit per time.

Furthermore, the information about the injection of a medicinal fluid may further include a medicinal fluid injection mode. The medicinal fluid injection mode may be a continuous mode in which a predetermined volume of medicinal fluid is continuously injected during an operating period from the initiation of the injection of a medicinal fluid to the termination thereof, a PCA mode in which a specific volume of medicinal fluid is injected in addition to the predetermined volume of medicinal fluid injected the continuous mode, or a PCEA mode which is similar to the PCA mode and in which the medicinal fluid is injected not via a vein but via an epidural space.

Furthermore, the information about the injection of a medicinal fluid may further include the units (mg, $\mu$g, or mL) of the drug included in the medicinal fluid, the LOT which is the time which ranges from the injection of a bolus up to the subsequent injection of another bolus and for which the bolus switch is locked, the LD which is the dose of bolus that is administered to a patient at the beginning of the injection of the medicinal fluid, the bolus rate, and the KVO rate that is intended to keep a vein open even after the completion of the injection of the medicinal fluid.

At step S20, the screen display unit 30 of the apparatus 1 for injecting a medicinal fluid displays the information about the injection of a medicinal fluid set at step S10.

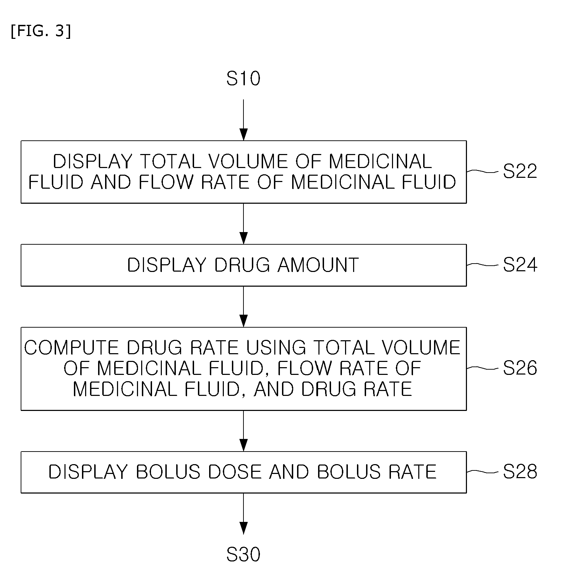

The details of step S20 will be described in detail with reference to FIG. 3.

At step S30, the apparatus 1 for injecting a medicinal fluid stores a history of the injection of the medicinal fluid that is injected into the patient in accordance with the information about the injection of a medicinal fluid set at step S10, for each predetermined time interval.

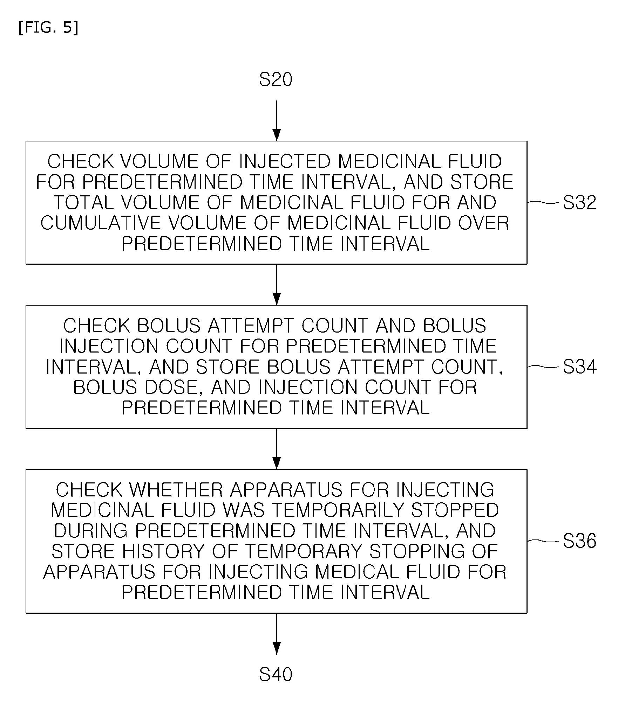

Here, the history of the injection of the medicinal fluid stored at S30 may include the volume of the injected medicinal fluid for the predetermined time interval, the cumulative volume of the injected medicinal fluid accumulated over the predetermined time interval, the bolus attempt count, the bolus injection count, and the bolus dose. The details of step S30 will be described in detail with reference to FIG. 5.

At step S40, the apparatus 1 for injecting a medicinal fluid analyzes the history of the injection of the medicinal fluid, stored at step S30, for the predetermined time interval, and regulates the status of the injection of the medicinal fluid into the patient.

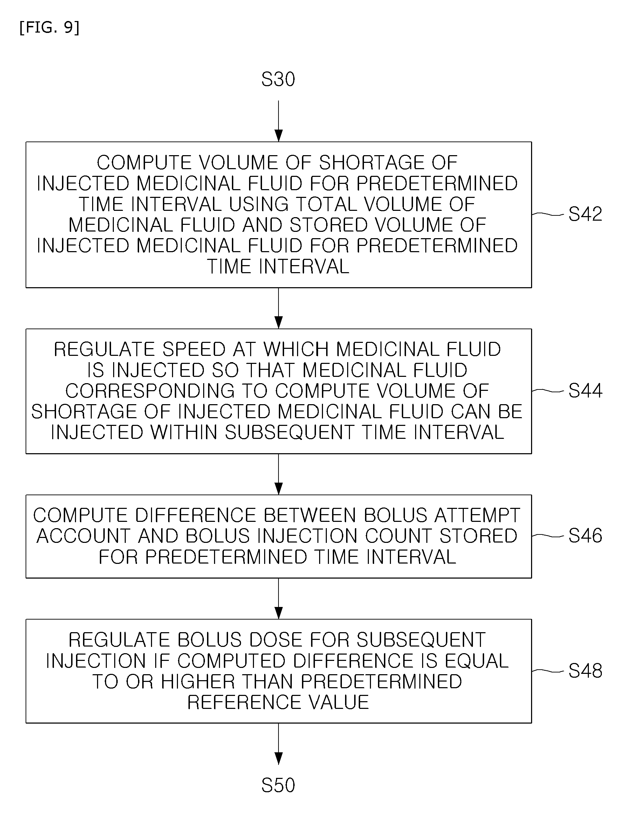

The details of step S40 will be described in detail with reference to FIG. 9.

At step S50, the apparatus 1 for injecting a medicinal fluid compares the cumulative volume of the injected medicinal fluid accumulated over the predetermined time interval with the total volume of the medicinal fluid set at step S10. Furthermore, the apparatus 1 for injecting a medicinal fluid terminates the process if the cumulative volume of the injected medicinal fluid accumulated over the predetermined time interval has reached the total volume of the medicinal fluid, and repeats steps S30 and S40 if the cumulative volume of the injected medicinal fluid accumulated over the predetermined time interval has not reached the total volume of the medicinal fluid.

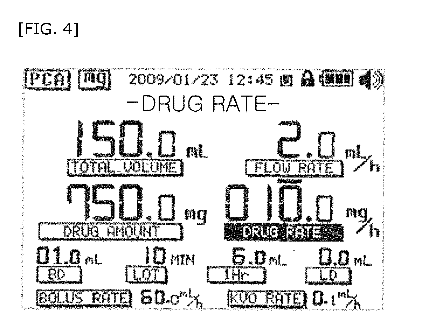

FIG. 3 is a detailed flowchart of step S20 of FIG. 2, and FIG. 4 is a reference diagram of the display of information about the injection of a medicinal fluid according to a preferred embodiment of the present invention.

As shown in FIGS. 3 and 4, at step S22, the screen display unit 30 of the apparatus 1 for injecting a medicinal fluid displays the total volume of the medicinal fluid and the flow rate of the medicinal fluid.

At step S24, the drug amount is displayed. At step S26, the drug rate is computed using the total volume of the medicinal fluid, the flow rate of the medicinal fluid, and the drug amount, and is then displayed.

Here, the drug rate may be computed using the following equation:

$$D_R = (D_A/T_V)*F_R \qquad\qquad (1)$$

where $D_R$ is the drug rate, $D_A$ is the drug amount, $T_V$ is the total volume of the medicinal fluid, and the $F_R$ is the flow rate of the medicinal fluid.

For example, if the drug amount is 750 mg, the total volume of the medicinal fluid is 150 mL, and the flow rate of the medicinal fluid is 2 mL/h, as shown in FIG. 4, the drug rate may be computed to be 10 mg/h based on Equation 1 and then displayed on the screen display unit 30 of the apparatus 1 for injecting a medicinal fluid.

Accordingly, even when the medicinal fluid is injected in drug units such as mg/h or μg/h, a user who uses the apparatus 1 for injecting a medicinal fluid according to the present invention can check the accurate drug rate on the screen display unit 30 of the apparatus 1 for injecting a medicinal fluid without any computational error attributable to direct computation.

At step S28, the screen display unit 30 of the apparatus 1 for injecting a medicinal fluid displays the bolus dose and the bolus rate, thereby terminating the present process.

In this case, at step S28, the screen display unit 30 of the apparatus 1 for injecting a medicinal fluid may further display the LOT, the LD, and the KVO rate.

FIG. 5 is a detailed flowchart of step S30 of FIG. 2.

As shown in FIG. 3, at step S32, the apparatus 1 for injecting a medicinal fluid checks the volume of the injected medicinal fluid for the predetermined time interval, and then stores the volume of the injected liquid for the predetermined time interval and the cumulative volume of the injected medicinal fluid accumulated over the predetermined time interval.

At step S34, the apparatus 1 for injecting a medicinal fluid checks the bolus attempt count and the bolus injection count for the predetermined time interval, and then stores a bolus dose, which is determined depending on the bolus attempt count and the bolus injection count, for the predetermined time interval.

At step S36, the apparatus 1 for injecting a medicinal fluid checks whether the apparatus for injecting a medicinal fluid was temporarily stopped during the predetermined time interval, and then stores a history of the temporary stopping of the apparatus for injecting a medicinal fluid for the predetermined time interval, thereby terminating the present process.

Here, the history of the pausing of the apparatus for injecting a medicinal fluid may include a case in which the apparatus 1 for injecting a medicinal fluid was stopped by the manipulation of the user, a case in which the apparatus 1 for injecting a medicinal fluid was stopped because the blockage of the medicinal fluid injection tube 50 was detected by the blockage sensor that detects the state of the blockage of the medicinal fluid injection tube 50, and a case in which the apparatus 1 for injecting a medicinal fluid was stopped because the opening of the door unit 50 was detected by the door sensor that detects the state of the opening and closing of the door unit 60.

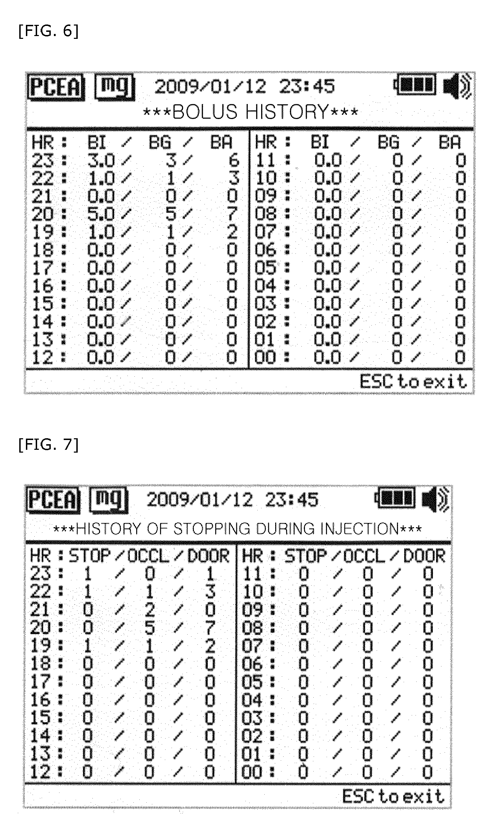

Furthermore, the screen display unit 30 of the apparatus 1 for injecting a medicinal fluid according to the preferred embodiment of the present invention displays general information about the injection of the medicinal fluid, the bolus history, and the history of the stopping of the apparatus for injecting a medicinal fluid stored for the predetermined time interval at step S30, which will be described with reference to FIGS. 6 to 8.

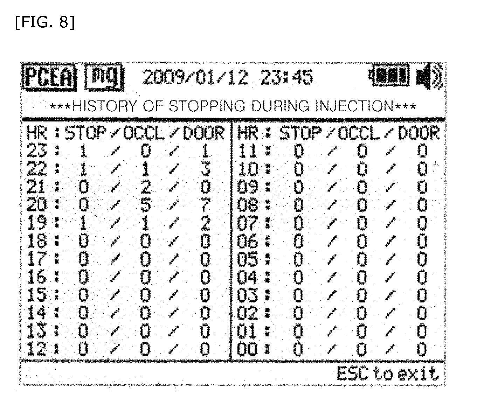

FIG. 6 of a reference screen of the display of general information about the injection of a medicinal fluid according to a preferred embodiment of the present invention, FIG. 7 is a reference screen of the display of a bolus history according to a preferred embodiment of the present invention, and FIG. 8 is a reference screen of a history of the stopping of the display apparatus for injecting a medicinal fluid according to a preferred embodiment of the present invention.

As shown in FIG. 6, the screen display unit 30 of the apparatus 1 for injecting a medicinal fluid according to the preferred embodiment of the present invention may display the general information about the injection of a medicinal fluid stored in the apparatus 1 for injecting a medicinal fluid.

Here, TOTAL denotes the total, 4HRLY denotes the predetermined time interval that can be regulated to one hour or four hours, SHIFT denotes the cumulative volume or count accumulated from a random point in time that is set by a user, Vol Inf denotes the volume of the injected medicinal fluid, Bol Inf denotes the bolus dose, Bol Given denotes the bolus injection count, Bol Attmp denotes the bolus attempt count, Clin Dose denotes the volume of injected clinical bolus, which is an additional bolus that can be injected in compliance with an instruction that was made by a doctor in charge depending on the state of a patient, Load Dose denotes the LD, and SHIFT Strt denotes the time at which SHIFT was reset by the user.

As shown in FIG. 7, the screen display unit 30 of the apparatus 1 for injecting a medicinal fluid according to the preferred embodiment of the present invention may display the bolus history stored in the apparatus 1 for injecting a medicinal fluid.

Here, HR denotes the predetermined time interval, BI denotes the bolus dose for the predetermined time interval, BG denotes the bolus injection count for the predetermined time interval, and BA denotes the bolus attempt count for the predetermined time interval.

As shown in FIG. 8, the screen display unit 30 of the apparatus 1 for injecting a medicinal fluid according to the preferred embodiment of the present invention may display the history of the stopping of the apparatus 1 for injecting a medicinal fluid stored in the apparatus 1 for injecting a medicinal fluid.

Here, HR denotes the predetermined time interval, STOP denotes the number of times that the apparatus 1 for injecting a medicinal fluid was stopped by the manipulation of the user during the predetermined time interval, OCCL denotes the number of times that the apparatus 1 for injecting a medicinal fluid was stopped for the predetermined time interval because the blockage of the medicinal fluid injection tube 50 was detected by the blockage sensor that detected the blockage of the medicinal fluid injection tube 50, and DOOR denotes the number of times that the apparatus 1 for injecting a medicinal fluid was stopped during the predetermined time interval because the opening of the door unit 50 was detected by the door sensor that detected the opening and closing of the door unit 60.

Accordingly, the user of the apparatus 1 for injecting a medicinal fluid according to the present invention can check the general information about the injection of a medicinal fluid, the bolus history, and the history of the stopping of the apparatus for injecting a medicinal fluid via the screen display unit 30, as shown in FIGS. 6 to 8, and can rapidly monitor the state of a patient as desired.

FIG. 9 is a detailed flowchart of step S40 of FIG. 2.

As shown in FIG. 9, at step S42, the apparatus 1 for injecting a medicinal fluid computes the volume of the shortage of the injected medicinal fluid for the predetermined time interval using the total volume of the medicinal fluid and the volume of the injected medicinal fluid stored for the predetermined time interval.

At step S44, the apparatus 1 for injecting a medicinal fluid regulates the speed at which the medicinal fluid is injected so that a medicinal fluid corresponding to the volume of the shortage of the injected medicinal fluid computed at step S42 can be injected within a subsequent predetermined time interval.

At step S46, the apparatus 1 for injecting a medicinal fluid computes the difference between the bolus attempt count and the bolus injection count stored for the predetermined time interval.

At step S48, the apparatus 1 for injecting a medicinal fluid regulates the bolus dose for the subsequent injection of the bolus if the difference computed at step S46 is equal to or higher than a predetermined reference value, thereby terminating the present process.

As described above, the apparatus 1 for injecting a medicinal fluid according to the present invention can analyze the history of the injection of a medicinal fluid, stored for the predetermined time interval, at the predetermined time interval and then cause a medicinal fluid corresponding to the volume of shortage to be injected within a subsequent time interval or regulate the bolus dose for a subsequent bolus injection, so that the user of the apparatus 1 for injecting a medicinal fluid according to the present invention can actively deal with the state of a patient based on the state of the patient by regulating the status of the injection of the medicinal fluid.

The method of managing a history of the injection of a medicinal fluid according to the present invention has the advantage of actively dealing with the state of a patient because information about the injection of a medicinal fluid can be set by manipulating the manipulation unit 20 of the apparatus 1 for injecting a medicinal fluid and then a history of the injection of a medicinal fluid injected into the patient based on the set information about the injection of the medicinal fluid can be stored for each predetermined time interval or the stored history of the injection of the medicinal fluid can be displayed on the screen display unit 30, and because the stored history of the injection of a medicinal fluid can be analyzed for the predetermined time interval and then the state of the injection of the medicinal fluid into the patient can be regulated.

Furthermore, the method of the present invention has the advantage of monitoring the state of a patient, into whom a medicinal fluid is being injected by the apparatus 1 for injecting a medicinal fluid, in real time because information about the injection of the medicinal fluid and the history of the injection of the medicinal fluid can be displayed on the screen display unit 30 of the apparatus 1 for injecting a medicinal fluid.

Furthermore, the method of the present invention has the advantage of reducing errors that may occur in a setting process, compared to a conventional stepwise display method, because information about the injection of the medicinal fluid and the history of the injection of the medicinal fluid can be displayed on the screen display unit 30 of the apparatus 1 for injecting a medicinal fluid in the form of a single screen.

The above description is merely an illustrative description of the technical spirit of the present invention, and those having ordinary knowledge in the technical field to which the present invention pertains can make a variety of modifications, variations, and substitutions within the range that does not depart from the essential characteristics of the present invention. Accordingly, the embodiments and attached drawings presented in conjunction with the present invention are intended not to limit the technical spirit of the present invention but to illustrate the technical spirit of the present invention. The scope of the technical spirit of the present invention is not limited by the embodiments and the attached drawings. The range of the protection of the present invention should be interpreted based on the following claims, and all technical spirits that fall within a range of equivalents should be interpreted as falling within the range of the rights of the present invention.

**Claims**

1. A method of managing a history of injection of a medicinal fluid in an apparatus for injecting a medicinal fluid, the method comprising the steps of:

   (a) setting information about injection of a medicinal fluid at an apparatus for injecting a medicinal fluid;
   (b) storing a history of injection of the medicinal fluid into a patient based on the set information about injection of the medicinal fluid for a predetermined time interval; and
   (c) analyzing the stored history of injection of the medicinal fluid for the predetermined time interval, and regulating a state of injection of the medicinal fluid into the patient.

2. The method of claim 1, wherein the information about injection of the medicinal fluid of step (a) comprises a total volume of the medicinal fluid, a flow rate of the medicinal fluid, an amount of drug included in the medicinal fluid, a bolus dose, a bolus rate, and a bolus limit per time.

3. The method of claim 2, further comprising, after step (a):

   step (a1) of displaying the information about injection of the medicinal fluid.

4. The method of claim 3, wherein step (a1) comprises the steps of:

   (a11) displaying the total volume of the medicinal fluid and the flow rate of the medicinal fluid;
   (a12) displaying the drug amount, computing a drug rate using the total volume of the medicinal fluid, the flow rate of the medicinal fluid, and the drug amount, and displaying the drug rate; and
   (a13) displaying the bolus dose and the bolus rate.

5. The method of claim 4, wherein the drug rate of step (a12) is computed using the following Equation:

$$D_R = (D_A/T_V)*F_R$$

   where $D_R$ is the drug rate, $D_A$ is the drug amount, $T_V$ is the total volume of the medicinal fluid, and the $F_R$ is the flow rate of the medicinal fluid.

6. The method of claim 2, wherein step (b) comprises the steps of:

   (b1) checking a volume of an injected medicinal fluid for the predetermined time interval, and storing the volume of the injected medicinal fluid and the total volume of the medicinal fluid for the predetermined time interval; and
   (b2) checking a bolus attempt count and a bolus injection count for the predetermined time interval, and storing the bolus attempt count, the bolus injection count, and the bolus dose.

7. The method of claim 6, further comprising, after step (b2):

   step (b3) of checking whether the apparatus for injecting a medicinal fluid was temporarily stopped during the predetermined time interval, and storing a history of the temporary stopping of the apparatus for injecting a medicinal fluid for the predetermined time interval.

8. The method of claim 7, wherein the volume of the injected medicinal fluid for the predetermined time interval and total volume of the medicinal fluid of step (b1), the bolus attempt count, bolus injection count and bolus dose of step (b2), and the history of the temporary stopping of step (b3) can be displayed on the apparatus for injecting a medicinal fluid.

9. The method of claim 6, wherein step (c) comprises the steps of:

   (c1) computing a volume of shortage of the injected medicinal fluid for the predetermined time interval using the total volume of the medicinal fluid and the volume of the injected medicinal fluid stored for the predetermined time interval; and
   (c2) regulating a speed at which the medicinal fluid is injected so that a medicinal fluid corresponding to the computed volume of the shortage of the injected medicinal fluid can be injected within a subsequent predetermined time interval.

10. The method of claim 9, further comprising, after step (c2):

   step (c3) of computing a difference between the bolus attempt count and the bolus injection count stored for the predetermined time interval, and regulating a bolus dose for subsequent bolus injection if the difference is equal to or higher than a predetermined reference value.

[FIG. 1]

[FIG. 2]

```
                    ┌──────────────┐
                    │    START     │
                    └──────────────┘
                           │
                           ▼
        ┌─────────────────────────────────────┐
        │  SET INFORMATION ABOUT INJECTION    │──── S10
        │       OF MEDICINAL FLUID            │
        └─────────────────────────────────────┘
                           │
                           ▼
        ┌─────────────────────────────────────┐
        │   DISPLAY SET INFORMATION ABOUT     │──── S20
        │     INJECTION OF MEDICINAL FLUID    │
        └─────────────────────────────────────┘
                           │
                           ▼
        ┌─────────────────────────────────────┐
        │    STORE HISTORY OF INJECTION OF    │──── S30
        │     MEDICINAL FLUID FOR EACH        │
        └─────────────────────────────────────┘
                           │
                           ▼
        ┌─────────────────────────────────────┐
        │      ANALYZE STORED HISTORY OF      │
        │  INJECTION OF MEDICINAL FLUID FOR   │
        │  PREDETERMINED TIME INTERVAL AND    │──── S40
        │   REGULATE STATUS OF INJECTION OF   │
        │    MEDICINAL FLUID INTO PATIENT     │
        └─────────────────────────────────────┘
                           │
                           ▼
```

NO

IS CUMULATIVE
VOLUME OF INJECTED
MEDICINAL FLUID EQUAL TO
OR LESS THAN TOTAL VOLUME
OF MEDICINAL FLUID? ──── S50

YES

END

[FIG. 3]

S10

DISPLAY TOTAL VOLUME OF MEDICINAL
FLUID AND FLOW RATE OF MEDICINAL FLUID — S22

DISPLAY DRUG AMOUNT — S24

COMPUTE DRUG RATE USING TOTAL VOLUME
OF MEDICINAL FLUID, FLOW RATE OF
MEDICINAL FLUID, AND DRUG RATE — S26

DISPLAY BOLUS DOSE AND BOLUS RATE — S28

S30

[FIG. 4]

[FIG. 5]

S20

```
                    │
                    ▼
┌──────────────────────────────────────────────┐
│  CHECK VOLUME OF INJECTED MEDICINAL FLUID     │
│  FOR PREDETERMINED TIME INTERVAL, AND STORE   │
│  TOTAL VOLUME OF MEDICINAL FLUID FOR AND      │─── S32
│  CUMULATIVE VOLUME OF MEDICINAL FLUID OVER    │
│  PREDETERMINED TIME INTERVAL                  │
└──────────────────────────────────────────────┘
                    │
                    ▼
┌──────────────────────────────────────────────┐
│  CHECK BOLUS ATTEMPT COUNT AND BOLUS          │
│  INJECTION COUNT FOR PREDETERMINED TIME       │
│  INTERVAL, AND STORE BOLUS ATTEMPT COUNT,     │─── S34
│  BOLUS DOSE, AND INJECTION COUNT FOR          │
│  PREDETERMINED TIME INTERVAL                  │
└──────────────────────────────────────────────┘
                    │
                    ▼
┌──────────────────────────────────────────────┐
│  CHECK WHETHER APPARATUS FOR INJECTING        │
│  MEDICINAL FLUID WAS TEMPORARILY STOPPED      │
│  DURING PREDETERMINED TIME INTERVAL, AND      │
│  STORE HISTORY OF TEMPORARY STOPPING OF       │─── S36
│  APPARATUS FOR INJECTING MEDICAL FLUID FOR    │
│  PREDETERMINED TIME INTERVAL                  │
└──────────────────────────────────────────────┘
                    │
                    ▼
```

S40

[FIG. 6]

[FIG. 7]

[FIG. 8]

[FIG. 9]

S30

↓

COMPUTE VOLUME OF SHORTAGE OF INJECTED MEDICINAL FLUID FOR PREDETERMINED TIME INTERVAL USING TOTAL VOLUME OF MEDICINAL FLUID AND STORED VOLUME OF INJECTED MEDICINAL FLUID FOR PREDETERMINED TIME INTERVAL — S42

↓

REGULATE SPEED AT WHICH MEDICINAL FLUID IS INJECTED SO THAT MEDICINAL FLUID CORRESPONDING TO COMPUTE VOLUME OF SHORTAGE OF INJECTED MEDICINAL FLUID CAN BE INJECTED WITHIN SUBSEQUENT TIME INTERVAL — S44

↓

COMPUTE DIFFERENCE BETWEEN BOLUS ATTEMPT ACCOUNT AND BOLUS INJECTION COUNT STORED FOR PREDETERMINED TIME INTERVAL — S46

↓

REGULATE BOLUS DOSE FOR SUBSEQUENT INJECTION IF COMPUTED DIFFERENCE IS EQUAL TO OR HIGHER THAN PREDETERMINED REFERENCE VALUE — S48

↓

S50